# EUROPÄISCHE PATENTSCHRIFT

(11) **EP 1 210 059 B1**
(45) Veröffentlichungstag und Bekanntmachung des Hinweises auf die Patenterteilung: **20.12.2006**
(21) Anmeldenummer: 01940306.2
(22) Anmeldetag: 07.04.2001
(51) Int. Cl.: A61K 8/02, A61Q 5/04, A61K 8/04

(54) **GELFÖRMIGES KOSMETISCHES MITTEL**
COSMETIC AGENT IN THE FORM OF A GEL
AGENT COSMETIQUE SOUS FORME DE GEL

(30) Priorität: 19.04.2000 DE 10019314
(43) Veröffentlichungstag der Anmeldung: 05.06.2002
(73) Patentinhaber: Wella Aktiengesellschaft, 64274 Darmstadt (DE)
(72) Erfinder: BIRKEL, Susanne, 95496 Glashütten (DE); ALLWOHN, Jürgen, 65558 Burgschwalbach (DE); WENDEL, Harald, 64372 Ober-Ramstadt (DE); FRANZKE, Michael, 64380 Rossdorf (DE); SCHREIBER, Birgit, 64678 Lindenfels (DE); KALBFLEISCH, Axel, 64295 Darmstadt (DE); STEINBRECHT, Karin, 64372 Ober-Ramstadt (DE)
(86) Internationale Anmeldenummer: PCT/EP2001/004024
(87) Internationale Veröffentlichungsnummer: WO 2001/080812

(56) Entgegenhaltungen:
- EP-A- 0 815 828
- WO-A-01/03658
- WO-A-92/07011
- WO-A-94/28877
- US-A- 5 626 154

## Beschreibung

Gegenstand der Erfindung ist ein kosmetisches Mittel in Form eines Gels mit einer speziellen Konsistenz und mit einer bestimmten Erscheinungsform der Oberfläche. Das Gel enthält ungelöste, gelierte, wassergequollene Polymerpartikel und bildet an der Grenzfläche zu gasförmigen Stoffen reversibel eine körnige, unebene, lichtstreuende Oberfläche und an der Grenzfläche zu festen Stoffen reversibel eine glatte, ebene, nicht lichtstreuende Oberfläche. Bevorzugte Gelbildner sind superabsorbierende Polymere. Gegenstand der Erfindung ist auch die Verwendung von superabsorbierenden Polymeren als Spitzenschutz bei Dauerwellbehandlungen.

Um dem menschlichen Haar Festigung und Halt zu geben oder um eine erstellte Frisur zu stabilisieren, werden Haarbehandlungsmittel u.a. in Form von verdickten Präparaten wie z.B. Gelen, Flüssig-Gelen, Sprüh-Gelen etc. eingesetzt. Derartige Produkte enthalten in der Regel eine Kombination aus Gelbildnern und haarfestigenden Polymeren. Die für diese Zwecke üblicherweise verwendeten kosmetischen, haarfestigenden Polymere zeigen in wässrigen, alkoholischen oder wässrig-alkoholischen Medien gute Festigungseigenschaften, die nach der Anwendung mehr oder weniger gut die Haare in Form halten und festigen und die erstellte Frisur stabilisieren. Nachteilig an herkömmlichen Haargelen ist der relativ hohe erforderliche Gehalt an Gelbildnern. Hierdurch sind höhere Rezepturkosten verbunden und es besteht eine verstärkte Gefahr von Inkompatibilitäten mit anderen Rezepturbestandteilen sowie von unerwünschten Nebeneffekten auf dem Haar wie z.B. Klebrigkeit, erhöhte Belastung etc.

Die der Erfindung zugrunde liegende Aufgabe bestand darin, kosmetische Mittel mit einerseits neuen, ausgefallenen Eigenschaften, insbesondere mit einer neuartigen äußeren Erscheinungsform oder einer neuartigen Konsistenz zur Verfügung zu stellen und andererseits die oben genannten Nachteile herkömmlicher Gele zu vermeiden, wobei die fachlichen Eigenschaften wie Haarfestigung, Haarstrukturgebung, Gel-Look oder Wet-Look beibehalten oder sogar verbessert werden. Eine charakteristische Eigenschaft eines Gels ist beispielsweise dessen Oberflächenbeschaffenheit. Durch die neuen, ausgefallenen Eigenschaften sollen die üblichen, kosmetischen Wirkungen allerdings nicht wesentlich beeinträchtigt, sondern idealerweise sogar noch verstärkt werden.

Zur Herstellung einer permanenten Haarverformung am menschlichen Haar, sind neben der Anwendung eines Reduktions- und Oxidationsmittels, Wickelkörper erforderlich. Das Haar wird auf seiner Längsachse von den Haarspitzen zum kopfhautnahen Ansatzbereich aufgerollt. Durch die mehrfache Umdrehung der Haare um den Wickler wird zwangsläufig die Haarspitze kleinlockiger als das Haar im Ansatzbereich. Die kleinlockige Spitzenumformung ist jedoch bei der Frisurenerstellung hinderlich und unerwünscht. Erschwerend kommt hinzu, dass das Haar im ungeschädigten (nativen) Zustand des Ansatzbereiches noch eine geschlossenere Cuticula aufweist. Dadurch ist der Ansatzbereich resistenter im Vergleich zu den um Monate älteren und durch den Einfluss des Kämmens, Waschens, Bleichens, Färbens oder Wellens und von Umwelteinflüssen zunehmend spröderen und durchlässigeren Spitzenbereich. Die zuvor erwähnten Nachteile bezüglich der Haarspitzen führen zu einer Verschlechterung der Struktur und der Frisierbarkeit.

Wiederholt wurde der Versuch unternommen, mittels Dauerwellvorbehandlungsmitteln (z. B. WO 97/09028) oder mit Säuren (z. B. DE 33 11 292 A und DE 1 492 007 A) oder Ölen (z. B. DE 42 36 726 A) getränktem Spitzenpapier ein gleichmäßiges Wellergebnis und einen Spitzenschutz oder Strukturausgleich zu erreichen. Ein mit Säuren oder mit Öl getränktes Spitzenpapier ist in seiner Wirkung bezüglich des Well- und Strukturausgleichs zu wirkungsschwach.

In der WO 92/07011 werden Haarbehandlungsmittel beschrieben mit einem Gehalt an stark quellbarem, leicht vernetztem Polyvinylpyrrolidon.

Aufgabe der vorliegenden Erfindung ist es, den oben genannten Überkrausungseffekt besser zu vermeiden, so dass während der Einwirkungszeit des Wellpräparates in besonderer Weise Längen und Spitzen gegenüber dem herkömmlichen Verfahren geschont werden und so die bekannten Nachteile vermieden werden. Damit sollte die Haarstruktur weniger geschädigt werden und die Längen und Spitzen einen vergleichbaren Wellenradius, wie der des kopfhautnahen Ansatzbereiches, trotz engerer Windungen, erhalten.

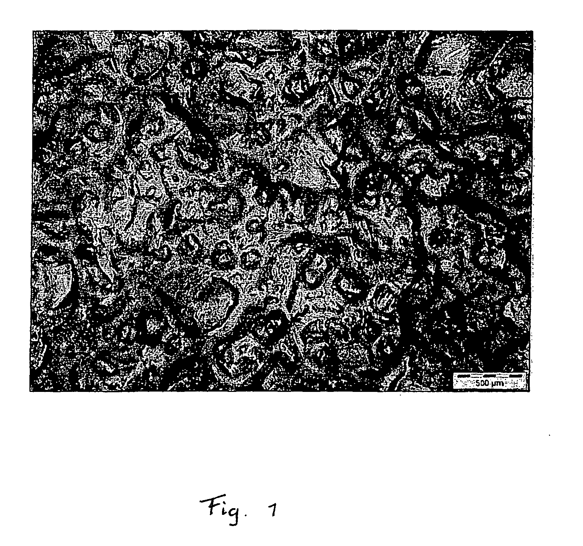

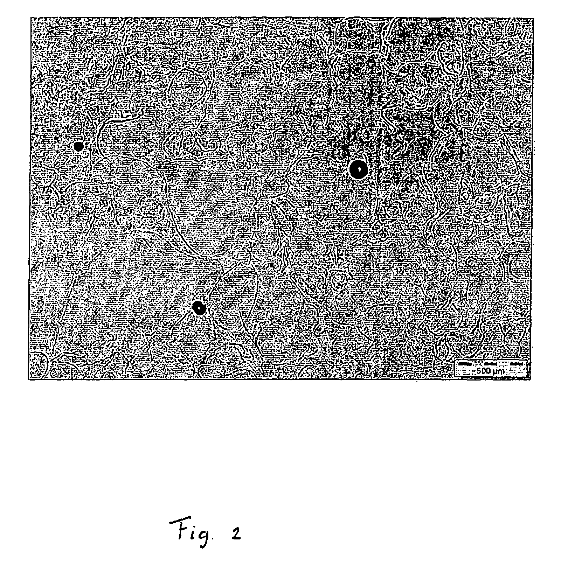

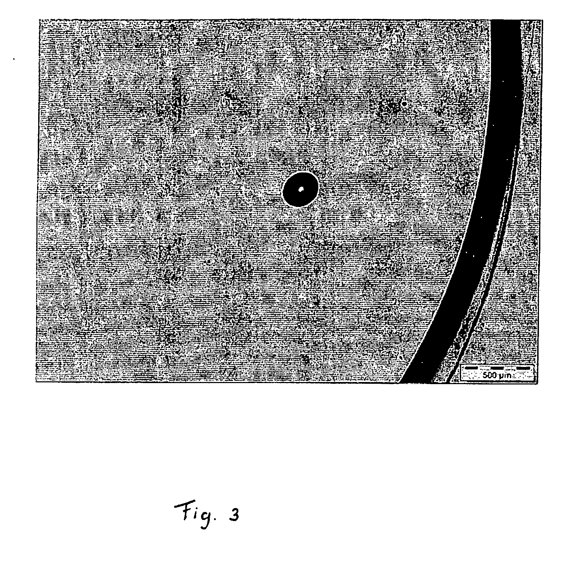

Es wurde nun gefunden, dass die Aufgabe gelöst wird durch ein gelförmiges kosmetisches Mittel, insbesondere ein Hydrogel, welches ungelöste, gelierte, wassergequollene Polymerpartikel enthält. Ungelöste Polymerpartikel im Sinne der Erfindung sind solche, welche im Unterschied zu herkömmlichen Gelbildnern (s. Abb. 3/3) auch im gequollenen Zustand nicht zerfließen sondern feste, diskrete Gelkörper bilden (s. Abb. 1/3 und 2/3). Das erfindungsgemäße Gel bildet an der Grenzfläche zu gasförmigen Stoffen reversibel eine körnige, unebene, lichtstreuende Oberfläche (s. Abb. 1/3) und an der Grenzfläche zu festen Stoffen reversibel eine glatte, ebene, nicht lichtstreuende Oberfläche (s. Abb. 2/3). Zur Bildung der Gelstruktur werden vorzugsweise wasserquellbare und wasserabsorbierende Polymere, insbesondere superabsorbierende Polymere eingesetzt. Die superabsorbierenden Polymere bilden bei Kontakt mit Wasser bzw. wässrigen Lösungen gequollene, gelierte Partikel, welche in dem erfindungsgemäßen Gel in dispergierter oder assoziierter Form vorliegen. Superabsorbierende Polymere sind bekannt durch ihre Verwendung in absorbierenden Sanitärartikeln wie Babywindeln, bei der Erwachseneninkontinenz, der Damenhygiene und der Wundabdeckung. Sie können definiert werden als wasserunlösliche, vernetzte Polymere, die in der Lage sind, unter Quellung und Ausbildung von Hydrogelen ein mehrfaches, d.h. bis zum 1000-fachen ihres Eigengewichts an wässrigen Flüssigkeiten aufzunehmen und die absorbierte Flüssigkeitsmenge unter Druck zurückzuhalten.

Gegenstand der Erfindung ist auch die Verwendung von superabsorbierenden Polymeren als Spitzenschutz bei Dauerwellbehandlungen. Überraschend wurde gefunden, dass es durch die Verwendung von superabsorbierenden Polymeren als Spitzenschutz gelingt, das unerwünschte und zu kleinlockige Wellergebnis in den Haarspitzen zu verhindern. Gleichzeitig wird der empfindliche Teil des Haares durch das Gel geschützt und durch örtliche Verdünnung der Wellflüssigkeit einer übermäßigen Quellung entgegen gewirkt. Sogar bei vorgeschädigtem Haar konnte unter Verwendung des gelförmigen kosmetischen Mittels ein einheitliches Wellbild erhalten werden.

Gegenstand der Erfindung ist auch ein Haarbehandlungsmittel in Form eines Gels mit einem Gehalt an wassergequollenen Polymerpartikeln aus wasserabsorbierenden, insbesondere superabsorbierenden Polymeren.

In einer besonderen Ausführungsform ist das Haarbehandlungsmittel ein Haarstyling- oder ein Haarpflegemittel mit einem Gehalt an
(A) mindestens einem superabsorbierenden Polymer und
(B) mindestens einem zweiten Polymer, welches ausgewählt ist aus haarfestigenden Polymeren und haarpflegenden Polymeren,
wobei das superabsorbierende Polymer (A) vorzugsweise in Form von ungelösten, wassergequollenen, gelierten Partikeln vorliegt.

Die erfindungsgemäßen Gele zeichnen sich durch ihre besondere äußere Erscheinungsform und Konsistenz aus. Die gelierten Partikel haben vorzugsweise einen mittleren Durchmesser von kleiner oder gleich 2000 µm, insbesondere von 20 bis 2000 µm, besonders bevorzugt von 40 bis 1400 µm. Die Partikelgrößen lassen sich gut durch skalierte mikroskopische Aufnahmen entweder im Auflicht oder im Durchlicht feststellen (s. Abbildungen 1/3 und 2/3).

Abbildung 1/3 zeigt eine fotographische Aufnahme der Oberfläche eines erfindungsgemäßen Gels (Beispiel 2) im Auflicht (Objektiv 2,5x). Das Gel befindet sich auf einem Objektträger.
Abbildung 2/3 zeigt eine fotographische Aufnahme eines erfindungsgemäßen Gels (Beispiel 2) im Durchlicht (Objektiv 2,5x). Das Gel befindet sich auf einem Objekträger mit aufgelegtem Deckgläschen. Die dünnen schwarzen Linien zeigen die Umrisse der gequollenen Polymerpartikel. Die kleinen, runden schwarzen Objekte sind Luftblasen.
Abbildung 3/3 zeigt eine fotographische Aufnahme eines herkömmlichen, nicht erfindungsgemäßen Gels (Vergleichsbeispiel) mit vollständig gelöstem Gelbildner im Durchlicht (Objektiv 2,5x). Das Gel befindet sich auf einem Objekträger mit aufgelegtem Deckgläschen. Die kleinen, runden schwarzen Objekte sind Luftblasen.

Die erfindungsgemäß einsetzbaren superabsorbierenden Polymere sind bekannt durch ihre Verwendung als Absorber für Flüssigkeiten, beispielsweise in Babywindeln. Hierbei handelt es sich üblicherweise um Polymere oder hydrophile Copolymere von Acrylsäure, Methacrylsäure oder um Pfropfcopolymere aus Stärke und Acrylsäure, wobei die Polymere neutralisiert oder teilneutralisiert als Salze vorliegen können. Sie werden gebildet durch Polymerisation unter teilweiser Vernetzung mit geeigneten Vernetzern aus ethylenisch ungesättigten hydrophilen Monomeren, insbesondere Acrylsäure, Methacrylsäure oder deren Alkalisalzen. Derartige Polymere und deren Herstellung sind vielfach beschrieben, exemplarisch wird auf die EP 0 312 952, die DE 44 18 818 und auf die EP 0 441 507 verwiesen. Besonders bevorzugt werden superabsorbierende Natriumpolyacrylate. Die superaborbierenden Polymere zeichnen sich durch ihr grosses Wasseraufnahmevermögen und ihr grosses Wasserrückhaltevermögen aus. Sie sind im Handel in Pulver- oder Granulatform erhältlich. Geeignete superabsorbierende Polymere sind beispielsweise AQUA-KEEP^{®} D (Elf Atochem S.A.), Sanwet^{®} IM 7015 (BASF AG), Sanwet^{®} 3746-5 (BASF AG), Hysorb^{®} E1290-00 (BASF AG), Hysorb^{®} E 1291-00 (BASF AG) oder Norsocryl XFS (Elf Atochem). Die mittlere Teilchengröße der trockenen Polymere beträgt vorzugsweise 100 bis 850 µm. Besonders bevorzug werden allerdings kleinere Teilchengrößen von 200 µm oder darunter. Das Aufnahmevermögen für entsalztes Wasser (Centrifuge Retention Capacity) liegt vorzugsweise bei mindestens 20 g/g. Die superabsorbierenden Polymere sind vorzugsweise in einer Menge von 0,05 bis 20 Gew.%, besonders bevorzugt von 0,1 bis 2 Gew.% enthalten und stellen vorzugsweise den einzigen Gelbildner des erfindungsgemäßen Gels dar. Zur Dauerwellvorbehandlung werden die superabsorbierenden Polymere vorzugsweise in einer Menge von 0,1 bis 5 Gew.%, besonders bevorzugt von 0,5 bis 2 Gew.% eingesetzt.

Das in der bevorzugten Ausführungsform enthaltene zweite Polymer (B) liegt vorzugsweise in einer Menge von 0,1 bis 30 Gew.%, besonders bevorzugt von 0,5 bis 15 Gew.% vor. Das zweite Polymer (B) ist ausgewählt aus haarpflegenden und haarfestigenden Polymeren. Dieses Polymer kann nichtionisch, anionisch, kationisch, zwitterionisch oder amphoter sein. Besonders bevorzugt sind nichtionische Polymere. Es kann ein synthetisches oder ein natürliches Polymer sein. Unter natürlichen Polymeren werden auch chemisch modifizierte Polymere natürlichen Ursprungs verstanden. Bevorzugt sind insbesondere solche Polymere, die eine ausreichende Löslichkeit oder Dispergierbarkeit in Wasser, Alkohol oder Wasser/Alkohol-Gemischen besitzen, um in dem erfindungsgemäßen Mittel in gelöster oder homogen dispergierter Form vorzuliegen. Unter haarfestigenden Polymeren werden erfindungsgemäß solche Polymere verstanden, die bei Anwendung in 0,01 bis 5%iger wässriger, alkoholischer oder wässrig-alkoholischer Lösung in der Lage sind, auf dem Haar einen Polymerfilm abzuscheiden. Unter haarpflegenden Polymeren werden solche Polymere verstanden, die eine Substantivität aufweisen, sich an das Haar anlagern und auf diese Weise eine Pflegewirkung auf das Haar ausüben, beispielsweise die Kämmbarkeit, den Griff oder den Glanz des Haares verbessern.

Besonders bevorzugt werden in dem erfindungsgemäßen Gel filmbildende, haarfestigende, nichtionische Polymere eingesetzt. Diese erwiesen sich im Gegensatz zu ionischen Polymeren in weiten Bereichen als besser kompatibel mit den superabsorbierenden Polymeren. Geeignete nichtionische Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, Alkyl- und Dialkylacrylamid, Alkyl- und Dialkylmethacrylamid, Alkylacrylat, Alkylmethacrylat, Propylenglykol oder Ethylenglykol, wobei die Alkylgruppen dieser Monomere vorzugsweise C1- bis C7-Alkylgruppen, besonders bevorzugt C1- bis C3-Alkylgruppen sind. Geeignet sind z.B. Homopolymere des Vinylcaprolactams, des Vinylpyrrolidons oder des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akypomine^{®} P 191 von der Firma CHEM-Y, Emmerich, oder Sepigel^{®} 305 von der Firma Seppic vertrieben werden; Polyvinylalkohole, die beispielsweise unter den Handelsbezeichnungen Elvanol^{®} von Du Pont oder Vinol^{®} 523/540 von der Firma Air Products vertrieben werden sowie Polyethylenglykol/Polypropylenglykol Copolymere, die beispielsweise unter den Handelsbezeichnungen Ucon^{®} der Union Carbide vertrieben werden. Besonders bevorzugt sind Polyvinylpyrrolidon, Polyvinylcaprolactam und deren Copolymere mit mindestens einem weiteren nichtionischen Monomer, insbesondere Polyvinylpyrrolidon/Vinylacetat Copolymere.

Das erfindungsgemäße Gel wird bevorzugt in einem wässrigen oder in einem wässrig-alkoholischen Medium mit vorzugsweise bis zu 30 Gew.% Alkohol konfektioniert. Der Alkoholgehalt beträgt besonders bevorzugt von 15 bis 20 Gew.%. In diesem Fall kann vorteilhafterweise auf den Einsatz von Konservierungsmitteln verzichtet werden. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Das erfindungsgemäße Mittel kann in einem pH-Bereich von 2,0 bis 9,5 vorliegen. Besonders bevorzugt ist der pH-Bereich zwischen 2,5 und 8. Als zusätzliche Co-Solventien können organische Lösungsmittel oder ein Gemisch aus Lösungsmitteln mit einem Siedepunkt unter 400°C in einer Menge von 0,1 bis 15 Gew.% bevorzugt von 1 bis 10 Gew.% enthalten sein. Besonders geeignet als zusätzliche Co-Solventien sind Glycerin, Ethylenglykol, Propylenglykol und Polyethylenglykole in einer Menge bis 30 Gew.%.

Gegebenenfalls kann die Konsistenz des erfindungsgemäßen Gels durch einen Gehalt an weiteren, üblichen Verdickern oder Gelbildnern optimiert werden. Hierfür geeignet sind z.B. Carboxyvinylpolymere, insbesondere Polyacrylate wie z.B. die verschiedenen Carbopol-Typen, außerdem Polyglykole, Cellulosederivate, insbesondere Hydroxyalkylcellulosen sowie Alginate, Carageenan und anorganische Verdicker wie z.B. natürliche oder synthetische Bentonite. Typische Einsatzkonzentrationen der zusätzlichen Gelbildner und Verdicker sind von etwa 0,2 bis 3,0 Gew.%, vorzugsweise von 0,2 bis 1 Gew.%.

Das erfindungsgemäße Mittel kann darüber hinaus die für Haarbehandlungsmittel üblichen Zusatzbestandteile enthalten, zum Beispiel Netzmittel oder Emulgatoren aus den Klassen der nichtionischen, anionischen, kationischen oder amphoteren oberflächenaktiven Tenside, wie ethoxylierte oder nicht ethoxylierte Fettalkoholsulfate, Alkylbenzolsulfonate, Alkyltrimethylammoniumsalze, Alkylbetaine, in einer Menge von 0,1 bis 15 Gew.%; Feuchthaltemittel; Parfümöle in einer Menge von 0,1 bis 0,5 Gew.%; Trübungsmittel, wie zum Beispiel Ethylenglykoldistearat, in einer Menge von etwa 0,2 bis 5,0 Gew.%; Perlglanzmittel, wie zum Beispiel ein Gemisch aus Fettsäuremonoalkylolamid und Ethylenglykoldistearat, in einer Menge von etwa 1,0 bis 10 Gew.%; bakterizide und fungizide Wirkstoffe wie zum Beispiel 2,4,4-Trichlor-2-hydroxydiphenylether oder Methylchlorisothiazolion, in einer Menge von 0,01 bis 1,0 Gew.%; Puffersubstanzen, wie beispielsweise Natriumcitrat oder Natriumphosphat, in einer Menge von 0,1 bis 1,0 Gew.%; Anfärbestoffe, wie zum Beispiel Fluorescein Natriumsalz, in einer Menge von etwa 0,1 bis 1,0 Gew.%; Pflegestoffe, wie zum Beispiel Pflanzen- und Kräuterextrakte, Protein- und Seidenhydrolysate, Lanolinderivate, in einer Menge von 0,1 bis 5 Gew.%; physiologisch verträgliche Silikonderivate, wie zum Beispiel flüchtige oder nicht-flüchtige Silikonöle oder hochmolekulare Siloxanpolymere in einer Menge von 0,05 bis 20 Gew.%; Lichtschutzmittel, Antioxidantien, Radikalfänger, Antischuppenwirkstoffe, in einer Menge von etwa 0,01 bis 2 Gew.%; Fettalkohole, Glanzgeber, Vitamine, Weichmacher, Kämmbarkeitsverbesserer, rückfettende Agenzien und Entschäumer.

Die Viskosität des erfindungsgemäßen Gels beträgt vorzugsweise von 500 bis 3000 mPa s, besonders bevorzugt von 1000 bis 1500 mPa s 25°C (gemessen mit einem Rotationsviskosimeter RheoStress 100 der Firma Haake bei einer Temperatur von 25°C und einem Schergefälle von 0,5 bis 1400 s⁻¹).

Zur Verwendung als Spitzenschutz bei Dauerwellbehandlungen können die beschriebenen Gele direkt mittels Pinsel oder Spatel auf die Haarspitzen aufgebracht werden. Auch das Auftragen eines Gelstranges aus einer Tube ist praktikabel. Alternativ können die Haarspitzen auch in das Gel getaucht oder über das Gel hinweg gezogen werden, so dass eine ausreichende Menge Gel haften bleibt. Auf die Spitzen der zum Dauerwellprozess abgeteilten Haarsträhnen wird jewels eine Menge von 0,5 bis 5 g Gel, bevorzugt 0,8 bis 3 g, aufgetragen. Es können auch flüssigkeitsdurchlässige Folien oder Spitzenpapiere als Hilfsmittel verwendet werden. Die mit dem erfindungsgemäßen Gel getränkten oder beschichteten Folien oder Spitzenpapiere sind ebenfalls Gegenstand der Erfindung. Folien wie Spitzenpapiere für die Dauerwelle sind an sich bekannt, sie bestehen üblicherweise aus nassfestem Papier, z. B. Langfaser-, Seiden- oder Japanpapier. Die Folie kann jedoch auch aus einem anderen saugfähigen Material wie z. B. Vliesstoff, Baumwollgewebe oder Gewebegemischen von Kunststofffasern mit Naturfasern bestehen. In der oben für das Haar beschriebenen Weise kann auch das Gel auf die Folie oder ein Spitzenpapier aufgetragen werden.

Vergleichsversuche an Probanden bei denen lediglich herkömmliches Spitzenpapier verwendet wurde, führten zu deutlich schwächeren und ungleichmäßigeren und daher unbefriedigenden Ergebnissen. Sie bestätigen somit sehr eindrucksvoll die oben aufgeführte Wirkung.

Die nachfolgenden Beispiele sollen den Gegenstand der Erfindung näher erläutern. Die in den Beispielen angegebenen Polymergehalte beziehen sich jeweils auf den Feststoffgehalt.

### Beispiele

### Beispiel 1: Kühlendes kosmetisches Mittel

| | |
|---|---|
| 0,5 g | Sanwet^{®} 3746-5 (BASF, Superabsorbierendes Natriumpolyacrylat) |
| 0,2 g | Menthol |
| 20 g | Ethanol |
| ad 100 g | Wasser |

### Beispiel 2: Haargel

| | |
|---|---|
| 0,7 g | Sanwet^{®} 3746-5 (BASF, Superabsorbierendes Natriumpolyacrylat) |
| 10 g | Luviskol^{®} VA 64 (BASF, PVP/VA Copolymer) |
| 0,2 g | PEG-40 Hydrogenated Castor Oil |
| 0,2 g | PPG-1-PEG-9 Lauryl Glykol Ether |
| 0,1 g | Parfüm |
| 20 g | Ethanol |
| ad 100 g | Wasser |

Abbildung 1/3 zeigt eine fotographische Aufnahme der Oberfläche des Gels im Auflicht (Objektiv 2,5x). Das Gel befindet sich auf einem Objektträger.
Abbildung 2/3 zeigt eine fotographische Aufnahme des Gels im Durchlicht (Objektiv 2,5x). Das Gel befindet sich auf einem Objekträger mit aufgelegtem Deckgläschen. Die dünnen schwarzen Linien zeigen die Umrisse der gequollenen Polymerpartikel. Die kleinen, runden schwarzen Objekte sind Luftblasen.

### Beispiel 3: Haargel

| | |
|---|---|
| 0,6 g | Sanwet^{®} 3746-5 (BASF, Superabsorbierendes Natriumpolyacrylat) |
| 12 g | Glucose |
| 0,2 g | PEG-40 Hydrogenated Castor Oil |
| 0,2 g | PPG-1-PEG-9 Lauryl Glykol Ether |
| 0,1 g | Parfüm |
| 15 g | Ethanol |
| ad 100 g | Wasser |

### Beispiel 4: Haargel

| | |
|---|---|
| 0,5 g | Sanwet^{®} 3746-5 (BASF, Superabsorbierendes Natriumpolyacrylat) |
| 10 g | Luviskol^{®} K30 (BASF, Polyvinylpyrrolidon) |
| 0,2 g | PEG-40 Hydrogenated Castor Oil |
| 0,2 g | PPG-1-PEG-9 Lauryl Glykol Ether |
| 0,1 g | Parfüm |
| 20 g | Ethanol |
| ad 100 g | Wasser |

### Beispiel 5: Haarglanzgel

| | |
|---|---|
| 0,7 g | Sanwet^{®} 3746-5 (BASF, Superabsorbierendes Natriumpolyacrylat) |
| 10 g | Glycerin |
| 0,2 g | PEG-40 Hydrogenated Castor Oil |
| 0,2 g | PPG-1-PEG-9 Lauryl Glykol Ether |
| 0,1 g | Parfüm |
| 20 g | Ethanol |
| ad 100 g | Wasser |

### Beispiel 6: Vergleichsbeispiel

| | |
|---|---|
| 0,8 g | Carbomer (vernetzter Polyacrylsäure-Verdicker) |
| 0,59 g | Aminomethylpropanol |
| 2,0 g | Polyvinylpyrrolidon |
| 1,72 g | Glycerin |
| 0,8 g | Polysorbate 40 |
| 0,2 g | PPG-1-PEG-9 Lauryl Glykol Ether |
| 0,2 g | Parfüm |
| 14,25 g | Ethanol |
| ad 100 g | Wasser |

Abbildung 3/3 zeigt eine fotographische Aufnahme des Vergleichgels mit vollständig gelöstem Gelbildner im Durchlicht (Objektiv 2,5x). Das Gel befindet sich auf einem Objekträger mit aufgelegtem Deckgläschen. Die kleinen, runden schwarzen Objekte sind Luftblasen.

### Beispiel 7: Stylinggel

| | |
|---|---|
| 0,7 g | Norsocryl^{®} XFS (Elf Atochem, superabsorbierendes Natriumpolyacrylat) |
| 7,5 g | Luviskol^{®} VA 64 (BASF, PVP/VA Copolymer) |
| 0,2 g | PEG-40 Hydrogenated Castor Oil |
| 0,2 g | PPG-1-PEG-9 Lauryl Glykol Ether |
| 0,1 g | Parfüm |
| 15 g | Ethanol |
| ad 100 g | Wasser |

### Beispiel 7: Spitzenschutz für Dauerwellbehandlung

| | |
|---|---|
| 2 g | Hysorb^{®} E1290-00 (BASF, Superabsorbierendes Natriumpolyacrylat) |
| ad 100 g | Wasser |

Normales, nicht vorgeschädigtes Haar wird gewaschen und mit einem Handtuch frottiert. Das durch Rühren oder Schütteln erhaltene Gel der oben genannten Zusammensetzung wird portionsweise (1g) auf die Spitzen der zu Strähnen abgeteilten Haare aufgetragen. Die Haare werden anschließend wie üblich auf Dauerwellwicker mit einem Durchmesser von 6 Millimetern gewickelt.

Anschließend wird das Haar mit dem folgenden Haarverformungsmittel gleichmäßig durchfeuchtet:

| | |
|---|---|
| 10,5 g | Thioglykolsäure, 80%ig |
| 5,0 g | Ammoniumhydrogencarbonat |
| 1,0 g | Ammoniak, 25%ig |
| 1,5 g | Kationisches Polymer |
| 1,0 g | Lösungsvermittler (Isooctylphenol, oxethyliert mit 10 Mol Ethylenoxid) |
| 0,4 g | Parfüm |
| ad 100 g | Wasser |

Nach einer Einwirkungszeit von 15 Minuten wird das Haar mit Wasser gründlich gespült und sodann mit 80 Gramm einer 3-prozentigen wäßrigen Hydrogenperoxid-Lösung oxidativ nachbehandelt. Nach Entfernung der Wickler werden die Haare erneut mit Wasser gespült, zur Wasserwelle gelegt und anschließend getrocknet. Nach Beendigung des Wellvorganges wird ein vom Haaransatz bis zur Haarspitze einheitliches Wellbild erhalten.

### Beispiel 8: Spitzenschutz für Dauerwellbehandlung

| | |
|---|---|
| 0,7 g | Sanwet^{®} 3746-5 (BASF, Superabsorbierendes Natriumpolyacrylat) |
| 0,2 g | PEG-40 Hydrogenated Castor Oil |
| 0,2 g | PPG-1-PEG-9 Lauryl Glykol Ether |
| 0,1 g | Parfüm |
| 20 g | Ethanol |
| ad 100 g | Wasser |

Mit dieser Zusammensetzung wird ein Dauerwellspitzenpapier beschichtet. Die Spitzen der zu behandelnden Haare werden strähnenweise in das Spitzenpapier eingelegt. Anschließend erfolgt die Dauerwellbehandlung wie bei Beispiel 7 beschrieben. Bei der Dauerwellbehandlung unter Verwendung des beschichteten Spitzenpapiers wird eine extreme Schonung der Haarspitzen erreicht, dessen Folge ein lang anhaltender, sanfter Wellenbogen des Haares ist.

### Beispiel 9: Spitzenschutz für Dauerwellbehandlung

| | |
|---|---|
| 1,2 g | Hysorb^{®} E1291-00 (BASF, Superabsorbierendes Natriumpolyacrylat) |
| 10 g | Glucose |
| 0,2 g | PEG-40 Hydrogenated Castor Oil |
| 0,2 g | PPG-1-PEG-9 Lauryl Glykol Ether |
| 0,1 g | Parfüm |
| 5 g | Ethanol |
| ad 100 g | Wasser |

Mit dieser Zusammensetzung wird ein Dauenivellspitzenpapier beschichtet. Die Spitzen der zu behandelnden Haare werden strähnenweise in das Spitzenpapier eingelegt. Anschließend erfolgt die Dauerwellbehandlung wie bei Beispiel 7 beschrieben. Bei der Dauerwellbehandlung unter Verwendung des beschichteten Spitzenpapiers wird eine extreme Schonung der Haarspitzen erreicht, dessen Folge ein lang anhaltender, sanfter Wellenbogen des Haares ist.

### Beispiel 10: Spitzenschutz für Dauerwellbehandlung

| | |
|---|---|
| 1,0 g | Sanwet^{®} 3746-5 (BASF, Superabsorbierendes Natriumpolyacrylat) |
| ad 100 g | Wasser |

Das durch Rühren oder Schütteln erhaltene Gel wird portionsweise (1g) auf die Spitzen der zu Strähnen abgeteilten Haare aufgetragen. Die Haare werden anschließend, wie bei Beispiel 7 beschrieben, auf Dauerwellwicker gewickelt und einer Dauerwellbehandlung unterworfen. Nach Beendigung des Wellvorganges wird ein vom Haaransatz bis zur Haarspitze einheitliches Wellbild erhalten.

### Beispiel 11: Vergleichtests

In einem Halbseitenversuch wurde das Haar auf der linken Kopfhälfte unter Verwendung von herkömmlichen Spitzenpapieren dem Dauerwellprozess unterzogen. Auf der rechten Kopfhälfte wurden wassergequollene Superabsorber

### Beispiel 12: Vergleichstest

In einem Halbseitenversuch wurde stark vorgeschädigtes Haar auf der linken Kopfhälfte unter Verwendung von herkömmlichen Spitzenpapieren dem Dauerwellprozess unterzogen. Auf der rechten Kopfhälfte wurden wassergequollene Superabsorber (Hysorb^{®} E1290-00), 1Gew. % in Wasser, als Spitzenschutz in der oben angegebenen Weise verwendet.

Sowohl im feuchten als auch im getrockneten Haar ist deutlich sichtbar, dass auf der rechten Kopfseite unter Verwendung des erfindungsgemäßen Gels ein gleichmäßigeres Wellbild vom Ansatz bis zu den Spitezen erhalten wird.

## Patentansprüche

1. Haarbehandlungsmittel in Form eines Gels mit einem Gehalt an wassergequollenen Polymerpartikeln aus superabsorbierenden Polymeren,
wobei die superabsorbierenden Polymere ein Wasseraufnahmevermögen für entsalztes Wasser (Centrifuge Retention Capacity) von mindestens 20 g/g haben und/oder wobei die superabsorbierenden Polymere ausgewählt sind aus superabsorbierenden vernetzten Polyacrylsäuren, Polymethacrylsäuren, Pfropfcopolymeren aus Stärke und Acrylsäure, wobei die Polymere auch neutralisiert oder teilneutralisiert als Salze vorliegen können.

2. Mittel nach Anspruch 1, **dadurch gekennzeichnet, dass** der mittlere Durchmesser der wassergequollenen Polymerpartikel kleiner oder gleich 2000 µm, vorzugsweise 40 bis 1400 µm beträgt.

3. Gelförmiges kosmetisches Mittel nach einem der vorhergehenden Ansprüche, **dadurch gekennzeichnet, dass** die superabsorbierenden Polymere vor Wasserabsorption eine mittlere Teilchengröße von 100 bis 850 µm, vorzugsweise von weniger als 200 µm haben.

4. Haarstyling- oder Haarpflegemittel nach Anspruch 1 mit einem Gehalt an mindestens einem zweiten Polymer, welches ausgewählt ist aus haarfestigenden Polymeren und haarpflegenden Polymeren.

5. Haarstyling- oder Haarpflegemittel nach Anspruch 4, **dadurch gekennzeichnet, dass** das Polymer (B) ausgewählt ist aus filmbildenden, haarfestigenden, nichtionischen Polymeren.

6. Haarstyling- oder Haarpflegemittel nach Anspruch 5, **dadurch gekennzeichnet, dass** das Polymer (B) ausgewählt ist Polyvinylpyrrolidon, Polyvinylcaprolactam und deren Copolymeren mit mindestens einem weiteren nichtionischen Monomer.

7. Haarstyling- oder Haarpflegemittel nach einem der Ansprüche 4 bis 6, **dadurch gekennzeichnet, dass** das superabsorbierende Polymer (A) in einer Menge von 0,05 bis 20 Gew.% enthalten ist.

8. Haarstyling- oder Haarpflegemittel nach einem der Ansprüche 4 bis 7, **dadurch gekennzeichnet, dass** das zweite Polymer (B) in einer Menge von 0,1 bis 30 Gew.% enthalten ist.

9. Verwendung eines gelförmigen kosmetischen Mittels enthaltend ungelöste, wassergequollene Polymerpartikel aus superabsorbierenden Polymeren als Spitzenschutz beim Dauerwellen von Haaren, wobei die superabsorbierenden Polymere ein Wasseraufnahmevermögen für entsalztes Wasser (Centrifuge Retention Capacity) von mindestens 20 g/g haben und/oder wobei die superabsorbierenden Polymere ausgewählt sind aus superabsorbierenden vernetzten Polyacrylsäuren, Polymethacrylsäuren, Pfropfcopolymeren aus Stärke und Acrylsäure, wobei die Polymere auch neutralisiert oder teilneutralisiert als Salze vorliegen können.

10. Verwendung nach Anspruch 9, **dadurch gekennzeichnet, dass** der mittlere Durchmesser der wassergequollenen Polymerpartikel kleiner oder gleich 2000 µm, vorzugsweise 40 bis 1400 µm beträgt.

11. Verwendung nach einem der Ansprüche 9 bis 11, **dadurch gekennzeichnet, dass** die superabsorbierenden Polymere vor Wasserabsorption eine mittlere Teilchengröße von 100 bis 850 µm, vorzugsweise kleiner 200 µm haben.

12. Verwendung nach einem der Ansprüche 9 bis 13 **dadurch gekennzeichnet, dass** das Mittel einen Feststoffgehalt an superabsorbierendem Polymer von 0,1 bis 5 Gew. % enthält.

13. Folie oder Papier zum Einwickeln von Haarspitzen, bei der Dauerwellbehandlungs **dadurch gekennzeichnet, dass** die Folie oder das Papier mit einer wässrigen Zubereitung, enthaltend mindestens ein superabsorbierendes Polymer in Form von wassergequollenen Polymerpartikeln getränkt oder beschichtet ist, wobei die superabsorbierenden Polymere ein Wasseraufnahmevermögen für entsalztes Wasser (Centrifuge Retention Capacity) von mindestens 20 g/g haben und/oder wobei die superabsorbierenden Polymere ausgewählt sind aus superabsorbierenden vernetzten Polyacrylsäuren, Polymethacrylsäuren, Pfropfcopolymeren aus Stärke und Acrylsäure, wobei die Polymere auch neutralisiert oder teilneutralisiert als Salze vorliegen können.

14. Verfahren zur dauerhaften Verformung von Haaren, bei dem man das Haar bevor und/oder nachdem man es in der gewünschten Form festhält, mit einem Verformungsmittel behandelt, mit Wasser spült, oxidativ nachbehandelt, erneut mit Wasser spült, gegebenenfalls zur Wasserwelle legt und sodann trocknet, **dadurch gekennzeichnet, dass** man die Haare vor der Behandlung mit dem Verformungsmittel mit einer Zusammensetzung behandelt, welche ungelöste, wassergequollene Polymerpartikel aus superabsorbierenden Polymeren enthält, wobei die superabsorbierenden Polymere ein Wasseraufnahmevermögen für entsalztes Wasser (Centrifuge Retention Capacity) von mindestens 20 g/g haben und/oder wobei die superabsorbierenden Polymere ausgewählt sind aus superabsorbierenden vernetzten Polyacrylsäuren, Polymethacrylsäuren, Pfropfcopolymeren aus Stärke und Acrylsäure, wobei die Polymere auch neutralisiert oder teilneutralisiert als Salze vorliegen können.

## Claims

1. Hair-treatment agent in the form of a gel with a content of water-swollen polymer particles of superabsorbent polymers, where the superabsorbent polymers have a water absorption capacity for demineralized water (centrifuge retention capacity) of at least 20 g/g and/or where the superabsorbent polymers are chosen from superabsorbent crosslinked polyacrylic acids, polymethacrylic acids, graft copolymers of starch and acrylic acid, where the polymers may also be present in neutralized or partially neutralized form as salts.

2. Agent according to Claim 1, **characterized in that** the average diameter of the water-swollen polymer particles is less than or equal to 2000 µm, preferably 40 to 1400 µm.

3. Cosmetic agent in the form of a gel according to one of the preceding claims, **characterized in that** the superabsorbent polymers have an average particle size of from 100 to 850 µm, preferably of less than 200 µm, before water absorption.

4. Hair-styling or haircare agent according to Claim 1 with a content of at least one second polymer which is chosen from hair-setting polymers and haircare polymers.

5. Hair-styling or haircare agent according to Claim 4, **characterized in that** the polymer (B) is chosen from film-forming, hair-setting, nonionic polymers.

6. Hair-styling or haircare agent according to Claim 5, **characterized in that** the polymer (B) is chosen from polyvinylpyrrolidone, polyvinylcaprolactam and copolymers thereof with at least one further nonionic monomer.

7. Hair-styling or haircare agent according to one of Claims 4 to 6, **characterized in that** the superabsorbent polymer (A) is present in an amount of from 0.05 to 20% by weight.

8. Hair-styling or haircare agent according to one of Claims 4 to 7, **characterized in that** the second polymer (B) is present in an amount of from 0.1 to 30% by weight.

9. Use of a cosmetic agent in the form of a gel comprising undissolved, water-swollen polymer particles of superabsorbent polymers as end protection during the permanent waving of hair, where the superabsorbent polymers have a water absorption capacity for demineralized water (centrifuge retention capacity) of at least 20 g/g and/or where the superabsorbent polymers are chosen from the superabsorbent crosslinked polyacrylic acids, polymethacrylic acids, graft copolymers of starch and acrylic acid, where the polymers may also be present in neutralized or partially neutralized form as salts.

10. Use according to Claim 9, **characterized in that** the average diameter of the water-swollen polymer particles is less than or equal to 2000 µm, preferably 40 to 1400 µm.

11. Use according to one of Claims 9 to 10, **characterized in that** the superabsorbent polymers have an average particle size of from 100 to 850 µm, preferably less than 200 µm, before water absorption.

12. Use according to one of Claims 9 to 11, **characterized in that** the agent comprises a solids content of superabsorbent polymer of from 0.1 to 5% by weight.

13. Foil or paper for winding hair ends during permanent waving treatment, **characterized in that** the foil or the paper is impregnated or coated with an aqueous preparation comprising at least one superabsorbent polymer in the form of water-swollen polymer particles, where the superabsorbent polymers have a water absorption capacity for demineralized water (centrifuge retention capacity) of at least 20 g/g and/or where the superabsorbent polymers are chosen from superabsorbent crosslinked polyacrylic acids, polymethacrylic acids, graft copolymers of starch and acrylic acid, where the polymers may also be present in neutralized or partially neutralized form as salts.

14. Method of permanently shaping hair, in which the hair, before and/or after it is held in the desired form, is treated with a shaping agent, rinsed with water, oxidatively after-treated, rinsed again with water, optionally set and then dried, **characterized in that** the hair, prior to treatment with the shaping agent, is treated with a composition which comprises undissolved, water-swollen polymer particles of superabsorbent polymers, where the superabsorbent polymers have a water absorption capacity for demineralized water (centrifuge retention capacity) of at least 20 g/g and/or where the superabsorbent polymers are chosen from superabsorbent crosslinked polyacrylic acids, polymethacrylic acids, graft copolymers of starch and acrylic acid, where the polymers may also be present in neutralized or partially neutralized form as salts.

## Revendications

1. Composition de traitement capillaire sous forme d'un gel ayant une teneur en particules de polymère gonflées dans l'eau, à base de polymères superabsorbants, les polymères superabsorbants ayant un pouvoir d'absorption d'eau, pour l'eau déminéralisée (*Centrifuge retention Capacity*), d'au moins 20 g/g et/ou les polymères superabsorbants étant choisis parmi des poly(acide acrylique)s, des poly(acide méthacrylique)s, des copolymères greffés d'amidon et acide acrylique, réticulés, superabsorbants, les polymères pouvant également se trouver neutralisés ou partiellement neutralisés sous forme de sels.

2. Composition selon la revendication 1, **caractérisée en ce que** le diamètre moyen des particules de polymère gonflées dans l'eau est inférieur ou égal à 2 000 µm, de préférence va de 40 à 1 400 µm.

3. Composition cosmétique sous forme de gel selon l'une quelconque des revendications précédentes, **caractérisée en ce que** les polymères superabsorbants ont avant l'absorption d'eau une taille moyenne de particule de 100 à 850 µm, de préférence de moins de 200 µm.

4. Composition de soin capillaire ou de coiffage selon la revendication 1, ayant une teneur en au moins un deuxième polymère qui est choisi parmi des polymères fixant les cheveux et des polymères de soin capillaire.

5. Composition de soin capillaire ou de coiffage selon la revendication 4, **caractérisée en ce que** le polymère (B) est choisi parmi des polymères non ioniques filmogènes, fixant les cheveux.

6. Composition de soin capillaire ou de coiffage selon la revendication 5, **caractérisée en ce que** le polymère (B) est choisi parmi la polyvinylpyrrolidone, le polyvinylcaprolactame et leurs copolymères avec au moins un autre monomère non ionique.

7. Composition de soin capillaire ou de coiffage selon l'une quelconque des revendications 4 à 6, **caractérisée en ce que** le polymère superabsorbant (A) est contenu en une quantité de 0,05 à 20 % en poids.

8. Composition de soin capillaire ou de coiffage selon l'une quelconque des revendications 4 à 7, **caractérisée en ce que** le deuxième polymère (B) est contenu en une quantité de 0,1 à 30 % en poids.

9. Utilisation d'une composition cosmétique sous forme de gel, contenant des particules de polymère non dissoutes, gonflées dans l'eau, à base de polymères superabsorbants, en tant que protection des pointes lors de l'ondulation permanente des cheveux, les polymères superabsorbants ayant un pouvoir d'absorption d'eau, pour l'eau déminéralisée (*Centrifuge retention Capacity*), d'au moins 20 g/g et/ou les polymères superabsorbants étant choisis parmi des poly(acide acrylique)s, des poly(acide méthacrylique)s, des copolymères greffés d'amidon et acide acrylique, réticulés, superabsorbants, les polymères pouvant également se trouver neutralisés ou partiellement neutralisés sous forme de sels.

10. Utilisation selon la revendication 9, **caractérisée en ce que** le diamètre moyen des particules de polymère gonflées dans l'eau est inférieur ou égal à 2 000 µm, de préférence va de 40 à 1 400 µm.

11. Utilisation selon l'une quelconque des revendications 9 à 10, **caractérisée en ce que** les polymères superabsorbants ont avant l'absorption d'eau une taille moyenne de particule de 100 à 850 µm, de préférence de moins de 200 µm.

12. Utilisation selon l'une quelconque des revendications 9 à 11, **caractérisée en ce que** la composition a une teneur en matière solide de polymère superabsorbant de 0,1 à 5 % en poids.

13. Film ou papier pour l'enroulement de pointes de cheveux lors du traitement d'ondulation permanente, **caractérisé en ce que** le film ou le papier est imprégné ou enduit d'une préparation aqueuse contenant au moins un polymère superabsorbant sous forme de particules de polymère gonflées dans l'eau, les polymères superabsorbants ayant un pouvoir d'absorption d'eau, pour l'eau déminéralisée (*Centrifuge retention Capacity*), d'au moins 20 g/g et/ou les polymères superabsorbants étant choisis parmi des poly(acide acrylique)s, des poly(acide méthacrylique)s, des copolymères greffés d'amidon et acide acrylique, réticulés, superabsorbants, les polymères pouvant également se trouver neutralisés ou partiellement neutralisés sous forme de sels.

14. Procédé pour la mise en forme permanente des cheveux, dans lequel les cheveux, avant et/ou après avoir été maintenus en la forme désirée, sont traités par une composition de mise en forme, rincés à l'eau, post-traités par oxydation, rincés de nouveau à l'eau, éventuellement mis en plis et ensuite séchés, **caractérisé en ce que**, avant le traitement par la composition de mis en forme, on traite les cheveux par une composition qui contient des particules de polymère non dissoutes, gonflées dans l'eau, à base de polymères superabsorbants, les polymères superabsorbants ayant un pouvoir d'absorption d'eau, pour l'eau déminéralisée (*Centrifuge retention Capacity*), d'au moins 20 g/g et/ou les polymères superabsorbants étant choisis parmi des poly(acide acrylique)s, des poly(acide méthacrylique)s, des copolymères greffés d'amidon et acide acrylique, réticulés, superabsorbants, les polymères pouvant également se trouver neutralisés ou partiellement neutralisés sous forme de sels.
